Europäisches Patentamt

European Patent Office

Office européen des brevets

(19)

(11) Publication number: **0 392 101**
**A1**

(12)

# EUROPEAN PATENT APPLICATION

(21) Application number: **89303665.7**

(22) Date of filing: **13.04.89**

(51) Int. Cl.5: **A61L 9/16**

The title of the invention has been amended (Guidelines for Examination in the EPO, A-III, 7.3).

(43) Date of publication of application:
**17.10.90 Bulletin 90/42**

(84) Designated Contracting States:
**AT BE CH DE ES FR GB GR IT LI LU NL SE**

(71) Applicant: **Chen, Ming Tse**
**315, Nanking W. Rd.**
**Taipei(TW)**

(72) Inventor: **Chen, Ming Tse**
**315, Nanking W. Rd.**
**Taipei(TW)**

(74) Representative: **Wells, Keith Raymond et al**
**POTTS, KERR & CO. 15 Hamilton Square**
**Birkenhead Merseyside L41 6BR(GB)**

(54) **Air purifier having a decorative housing.**

(57) A decorative assembly combining together an air purification apparatus (3) and a decorative housing therefor in which the decorative housing includes a trunk portion (1) having a hollow interior (2) whereby air is drawn from one end of the hollow interior (2) through the air purification apparatus (3) and is delivered from the other end (201) of the hollow interior (2).

*FIG. 1*

Xerox Copy Centre

## AIR PURIFIER HAVING A DECORATIVE HOUSING

The present invention relates to an air purifier having a decorative housing.

Air purification apparatuses are often installed in rooms in order to maintain the air in a pleasant state and to remove tobacco smoke and other malodorous particles. The air purification apparatus is usually contained in a casing which can either be located on the ground or hung on a wall. Such apparatuses however, are often unsuitable for use in restaurants and hotels. Firstly such places are often luxuriously decorated and furnished so as to provide an attractive ambience and this effect will inevitably be ruined by the intrusiveness of a functional air purification apparatus located on the floor or hanging on a wall. Moreover, such an apparatus is reluctantly used, a relatively small air purification apparatus which is less obtrusive will not effectively purify the air in a relatively large room and a relatively large air purification apparatus cannot be hidden. Still further the location of an air purification apparatus on the floor does not provide satisfactory air purification because the air inlet port and outlet port are both disposed at a relatively low level.

The present invention therefore seeks to provide an air purification apparatus having a decorative housing in which the housing participates in the purification process.

According to the present invention, there is provided an air purification apparatus having a decorative housing including a trunk portion characterized in that the trunk portion has a hollow interior in which is received an air purification apparatus, the trunk portion having opposed first and second end regions whereby air is drawn through one of said end regions into the hollow interior, is purified by the air purification apparatus and is discharged, in a purified form from the other of said end regions of the hollow interior.

The invention will be further described, by way of example, with reference to the accompanying drawings, in which:

Fig. 1 is an elevational view, partially in section, of a preferred embodiment of an air purification apparatus having a decorative housing in accordance with the present invention;

Fig. 2 is an exploded view of parts of the purification apparatus shown in Fig. 1; and

Fig. 3 is a vertical sectional view of the air purification apparatus having a decorative housing shown in Fig. 1 but on an enlarged scale relative thereto.

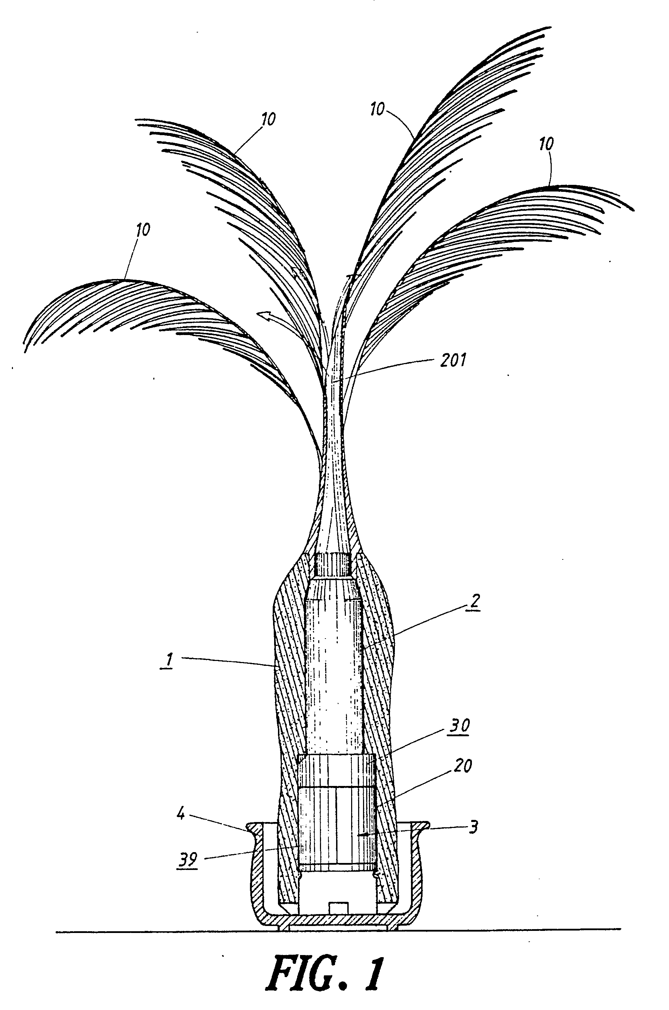

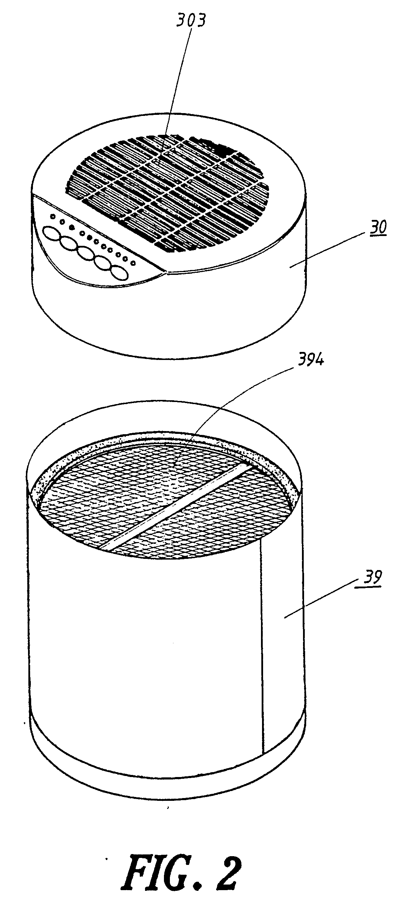

In the drawings, there is shown an air purification apparatus 3 having a decorative housing. The housing is, as shown, in the form of a palm tree and includes a trunk portion 1 made of, for example, polyethylene, polystyrene polyvinylchloride or polyurethane and branches and leaves 10 made of, for example, polyethylene or polyvinylchloride. The trunk portion 1 need not extend vertically and the artificial decoration need not necessarily be in the form of an artificial tree as shown in Fig. 1. The trunk portion 1 includes a hollow interior 2 having a lower end, an upper end 201, and a chamber 20 in which the air purification apparatus 3 is disposed. The air purification apparatus can be a two-part structure as shown in Fig. 2. In such a case, the upper portion 30 of the apparatus comprises a motor 301, a fan 302, upper ventilation apertures 303 and lower ventilation apertures 304. The lower portion 39 comprises a plurality of filtering screens which include a sponge layer 391, a fibrous layer 392, two active-carbon layers 393 and 394, and apertured top and bottom members. The screens 391 to 394 are suitably vertically spaced apart within the hollow interior 2 and can be resiliently mounted on the wall defining the hollow centre 2. The upper and lower portions 30, 39 can be interconnected by any suitable means. The air purification apparatus 3 can also be a unitary structure and alternative and/or additional features may be incorporated therein for an air sterilization, deodorization and/or dust removal purposes.

The air purification apparatus 3 may be actuated electrically using a conventional switch and wiring provided in the trunk portion 1 or may be actuated by a remote control device.

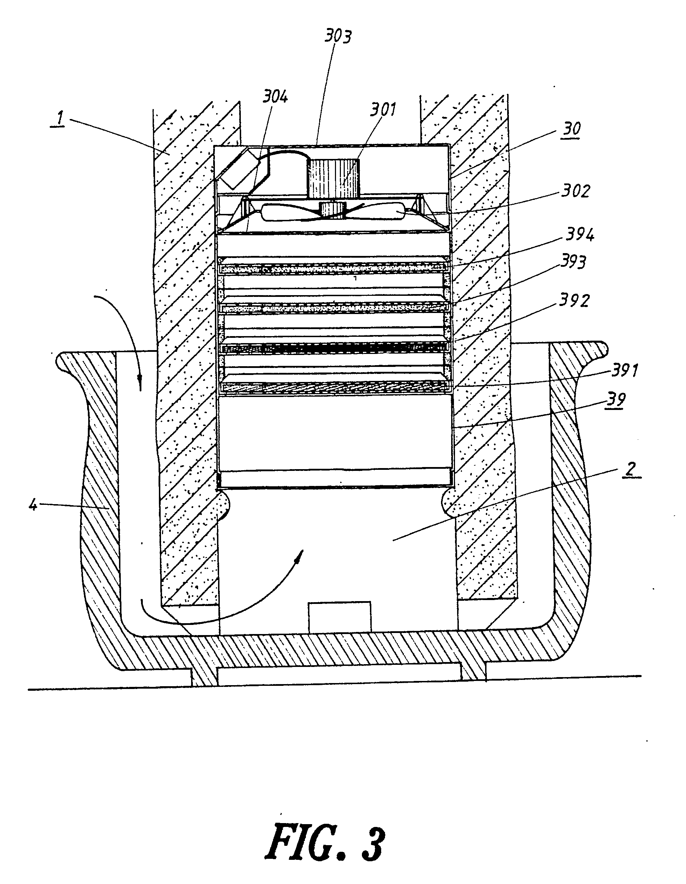

The air purification apparatus can be located in a suitably shaped base member 4 which draws air into the lower end of the hollow interior 2 in the manner shown in Fig. 3. Alternatively, if the lower end of the hollow interior 2 is closed by a support member transverse throughbores (not shown) may be provided in the trunk 1 which communicate with the lower end of the hollow interior 2.

After the motor 301 has been actuated, the rotating fan 302 continuously draws air from the lower end of the hollow interior 2 through the screens 391 to 394 whereby the air is purified and fresh air is returned to the ambient atmosphere through the upper end 201 of the trunk member 1.

As stated above, the decorative housing need not be in the form of an artificial tree and can be of any suitable decorative shape. Such an apparatus can be differently positioned in a room to provide both a decorative function and an air purification function simultaneously.

**Claims**

1. An air purification apparatus having a decorative housing including a trunk portion characterized in that the trunk portion has a hollow interior in which is received an air purification apparatus, the trunk portion having opposed first and second end regions whereby air is drawn through one of said end regions into the hollow interior is purified by the air purification apparatus and is discharged, in a purified form from the other of said end regions of the hollow interior.

2. An air purification apparatus as claimed in claim 1 characterized in that the decorative housing is in the form of an artificial tree.

3. An air purification apparatus as claimed in claim 1 or 2 characterized in that said decorative housing extends vertically and said first end of said hollow interior is the lower end thereof.

4. An air purification apparatus as claimed in any preceding claim characterized in that said trunk portion further includes transversely extending throughbores holes communicating with said first end.

5. An air purification apparatus as claimed in any preceding claim characterized in that said air purification apparatus is electrically actuated by a manually-operated switch.

6. An air purification apparatus as claimed in any one of claims 1 to 4, characterized in that said air cleaning apparatus is actuated by a remote-control device.

FIG. 1

*303*

*30*

*394*

*39*

# FIG. 2

FIG. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 732 591 (Y. TUJISAWA)<br>* Whole document *<br>----- | 1-6 | A 61 L 9/16 |
| | | | TECHNICAL FIELDS SEARCHED (Int. Cl.5) |
| | | | A 61 L |
| | The present search report has been drawn up for all claims | | |

| Place of search | Date of completion of the search | Examiner |
|---|---|---|
| THE HAGUE | 05-12-1989 | PELTRE CHR. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 
& : member of the same patent family, corresponding
    document

EPO FORM 1503 03.82 (P0401)